# EUROPÄISCHE PATENTANMELDUNG

(11) **EP 1 175 898 A2**
(43) Veröffentlichungstag der Anmeldung: **30.01.2002**
(21) Anmeldenummer: 01117424.0
(22) Anmeldetag: 19.07.2001
(51) Int. Cl.: A61K 7/48

(54) **Kosmetische oder dermatologische Verwendung von Kombinationen mit einem Gehalt an Carnitinen**

(30) Priorität: 28.07.2000 DE 10036797
(71) Anmelder: Beiersdorf Aktiengesellschaft, 20245 Hamburg (DE)
(72) Erfinder: Stäb, Dr. Franz, 21379 Echem (DE); Schreiner, Dr. Volker, 20259 Hamburg (DE); Sauermann, Dr. Gerhard, 24649 Wiemersdorf (DE)

(57) **Zusammenfassung**

Kosmetische oder dermatologische Zubereitungen mit einem Gehalt an einer Verbindung oder mehrerer Verbindungen aus der Gruppe A, gebildet von Carnitin und/oder Acyl-Carnitinen und jeweils deren Derivaten in Kombination mit einer Verbindung oder mehreren Verbindungen aus der Gruppe B, gebildet von Biotin, Liponsäure, konjugierten Fettsäuren, Carnosin, Biochinonen, Phytofluen, Phytoen und Folsäure und jeweils deren Derivaten, gegebenenfalls mit einem zusätzlichen Gehalt an einer Verbindung oder mehreren Verbindungen aus der Gruppe der Antioxidantien und/oder Energiestoffwechsel-Metaboliten, wobei Zubereitungen mit einem Gehalt an Acyl-Carnitinen oder deren Derivaten mindestens eine Verbindung aus einer Gruppe enthalten, die von Biotin, konjugierten Fettsäuren, Biochinonen, Phytofluen, Phytoen und jeweils deren Derivaten gebildet wird.

## Beschreibung

Die vorliegende Erfindung betrifft kosmetische bzw. dermatologische, insbesondere topische Zubereitungen, enthaltend Wirkstoffe zur Pflege und zum Schutze der Haut, insbesondere der empfindlichen Haut wie auch ganz besonders im Vordergrunde stehend der durch intrinsische und/oder extrinsische Faktoren gealterten oder alternden Haut sowie die Verwendung solcher Wirkstoffe und Kombinationen solcher Wirkstoffe auf dem Gebiete der kosmetischen und dermatologischen Hautpflege, zum Schutz vor Hautalterungsphänomenen (Falten, Schlaffheit, Puffiness, Energieverlust), zur Behandlung von Hautalterungsphänomenen, zur Vitalisierung, Regenerationssteigerung, zur Behandlung von degenerativen und entzündlichen Hauterscheinungen, zur Steigerung der Widerstandskraft gegen Umweltnoxen.

Unter kosmetischer Hautpflege ist in erster Linie zu verstehen, daß die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es femer, den durch tägliche Waschen verursachten Fett-und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Die chronologische Hautalterung wird z.B. durch endogene, genetisch determinierte Faktoren verursacht. In Epidermis und Dermis kommt es alterungsbedingt z.B. zu folgenden Strukturschäden und Funktionsstörungen, die auch unter den Begriff "Senile Xerosis" fallen können:
a) Trockenheit, Rauhigkeit und Ausbildung von Trockenheitsfältchen,
b) Juckreiz und
c) verminderte Rückfettung durch Talgdrüsen (z.B. nach Waschen).
   Exogene Faktoren, wie UV-Licht und chemische Noxen, können kumulativ wirksam sein und z.B. die endogenen Alterungsprozesse beschleunigen bzw. sie ergänzen. In Epidermis und Dermis kommt es insbesondere durch exogene Faktoren z.B. zu folgenden Strukturschäden- und Funktionsstörungen in der Haut, die über Maß und Qualität der Schäden bei chronologischer Alterung hinausgehen:
d) Sichtbare Gefäßerweiterungen (Teleangiektasien, Cuperosis);
e) Schlaffheit und Ausbildung von Falten;
f) lokale Hyper-, Hypo- und Fehlpigmentierungen (z.B. Altersflecken) und
g) vergrößerte Anfälligkeit gegenüber mechanischem Stress (z.B. Rissigkeit).

Die vorliegende Erfindung betrifft insbesondere Produkte zur Pflege der auf natürliche Weise gealterten Haut, sowie zur Behandlung der Folgeschäden der Lichtalterung, insbesondere der unter a) bis g) aufgeführten Phänomene.

Produkte zur Pflege gealterter Haut sind an sich bekannt. Sie enthalten z.B. Retinoide (Vitamin A-Säure und/oder deren Derivate) bzw. Vitamin A und/oder dessen Derivate. Ihre Wirkung auf die Strukturschäden ist allerdings umfangsmäßig begrenzt. Darüber hinaus gibt es bei der Produktentwicklung erhebliche Schwierigkeiten, die Wirkstoffe in ausreichendem Maße gegen oxidativen Zerfall zu stabilisieren. Die Verwendung Vitamin A-Säure-haltlger Produkte bedingt darüber hinaus oft starke erythematöse Hautreizungen. Retinoide sind daher nur in geringen Konzentrationen einsetzbar.

Insbesondere betrifft die vorliegende Erfindung kosmetische Zubereitungen mit einem wirksamen Schutz vor schädlichen Oxidationsprozessen in der Haut, aber auch zum Schutze kosmetischer Zubereitungen selbst bzw. zum Schutze der Bestandteile kosmetischer Zubereitungen vor schädlichen Oxidationsprozessen.

Die vorliegende Erfindung betrifft ferner Antioxidantien, bevorzugt solche, welche in hautpflegenden kosmetischen oder dermatologischen Zubereitungen eingesetzt werden. Insbesondere betrifft die Erfindung auch kosmetische und dermatologische Zubereitungen, solche Antioxidantien enthaltend. In einer bevorzugten Ausführungsform betrifft die vorliegende Erfindung kosmetische und dermatologische Zubereitungen zur Prophylaxe und Behandlung kosmetischer oder dermatologischer Hautveränderungen wie z.B. der Hautalterung, insbesondere der durch oxidative Prozesse hervorgerufenen Hautalterung.

Weiterhin betrifft die vorliegende Erfindung Wirkstoffe und Zubereitungen, solche Wirkstoffe enthaltend, zur kosmetischen und dermatologischen Behandlung oder Prophylaxe erythematöser, entzündlicher, allergischer oder autoimmunreaktiver Erscheinungen, insbesondere Dermatosen.

Die vorliegende Erfindung betrifft in einer weiteren vorteilhaften Ausführungsform Wirkstoffkombinationen und Zubereitungen, die zur Prophylaxe und Behandlung der lichtempfindlichen Haut, insbesondere von Photodermatosen, dienen.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutz gegen UVB-Strahlung sind zahlreiche Verbindungen bekannt, bei denen es sich um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, des sogenannten UVA-Bereich, ist es wichtig, Filtersubstanzen zur Verfügung zu haben, da dessen Strahlen Reaktionen bei lichtempfindlicher Haut hervorrufen können. Es ist erwiesen, daß UVA-Strahlung zu einer Schädigung der elastischen und kollagenen Fasern des Bindegewebes führt, was die Haut vorzeitig altem lässt, und daß sie als Ursache zahlreicher phototoxischer und photoallergischer Reaktionen zu sehen ist. Der schädigende Einfluss der UVB-Strahlung kann durch UVA-Strahlung verstärkt werden.

Zum Schutz gegen die Strahlen des UVA-Bereichs werden daher gewisse Derivate des Dibenzoylmethans verwendet, deren Photostabilität (Int. J. Cosm. Science 10, 53 (1988)), nicht in ausreichendem Maße gegeben ist.

Die UV-Strahlung kann aber auch zu photochemischen Reaktionen führen, wobei dann die photochemischen Reaktionsprodukte in den Hautmetabolismus eingreifen.

Vorwiegend handelt es sich bei solchen photochemischen Reaktionsprodukten um radikalische Verbindungen, beispielsweise Hydroxyradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Femer zählt UV-Strahlung zur ionisierenden Strahlung. Es besteht also das Risiko, daß auch ionische Spezies bei UV-Exposition entstehen, welche dann ihrerseits oxidativ in die biochemischen Prozesse einzugreifen vermögen.

Um diesen Reaktionen vorzubeugen, können den kosmetischen bzw. dermatologischen Formulierungen zusätzliche Antioxidantien und/oder Radikalfänger einverleibt werden.

Es ist bereits vorgeschlagen worden, Vitamin E, eine Substanz mit bekannter anti-oxidativer Wirkung in Lichtschutzformulierungen einzusetzen, dennoch bleibt auch hier die erzielte Wirkung weit hinter der erhofften zurück.

Aufgabe der Erfindung war es daher auch, kosmetische, dermatologische und pharmazeutische Wirkstoffe und Zubereitungen sowie Lichtschutzformulierungen zu schaffen, die zur Prophylaxe und Behandlung lichtempfindlicher Haut, insbesondere Photodermatosen, bevorzugt PLD dienen.

Weitere Bezeichnungen für die polymorphe Lichtdermatose sind PLD, PLE, Mallorca-Akne und eine Vielzahl von weiteren Bezeichnungen, wie sie in der Literatur (z.B. A. Voelckel et al, Zentralblatt Haut- und Geschlechtskrankheiten (1989), 156, S.2), angegeben sind.

Erythematöse Hauterscheinungen treten auch als Begleiterscheinungen bei gewissen Hauterkrankungen oder -unregelmäßigkeiten auf. Beispielsweise ist der typische Hautausschlag beim Erscheinungsbild der Akne regelmäßig mehr oder weniger stark gerötet.

Aus der DE-A 198 06 889 ist die Verwendung von bestimmten Acylcarnitinen zur Behandlung und Prophylaxe von Symptomen der intrinsischen und extrinsischen Hautalterung bekannt.

Hauptsächlich werden Antioxidantien als Schutzsubstanzen gegen den Verderb der sie enthaltenden Zubereitungen verwendet. Dennoch ist bekannt, daß auch in der menschlichen und tierischen Haut unerwünschte Oxidationsprozesse auftreten können. Solche Prozesse spielen eine wesentliche Rolle bei der Hautalterung.

Im Aufsatz "Skin Diseases Associated with Oxidative Injury" in "Oxidative Stress in Dermatology", S. 323 ff. (Marcel Decker Inc., New York, Basel, Hong Kong, Herausgeber: Jürgen Fuchs, Frankfurt, und Lester Packer, Berkeley/Californien), werden oxidative Schäden der Haut und ihre näheren Ursachen aufgeführt.

Auch aus dem Grunde, solchen Reaktionen vorzubeugen, können kosmetischen oder dermatologischen Formulierungen zusätzlich Antioxidantien und/oder Radikalfänger einverleibt werden.

Zwar sind einige Antioxidantien und Radikalfänger bekannt. So ist bereits in den US-Patentschriften 4,144,325 und 4,248,861 sowie aus zahlreichen anderen Dokumenten vorgeschlagen worden, Vitamin E, eine Substanz mit bekannter antioxidativer Wirkung in Lichtschutzformulierungen einzusetzen, dennoch bleibt auch hier die erzielte Wirkung weit hinter der erhofften zurück.

Aufgabe der vorliegenden Erfindung war es somit, Wege zu finden, die die Nachteile des Standes der Technik vermeiden. Insbesondere soll die Wirkung der Behebung der mit der endogenen, chronologischen und exogenen Alterung der Haut und der Hautanhangsgebilde verbundenen Schäden und die Prophylaxe dauerhaft, nachhaltig und ohne das Risiko von Nebenwirkungen sein.

Diesen Übelständen abzuhelfen, war Aufgabe der vorliegenden Erfindung.

Diese Aufgaben werden erfindungsgemäß gelöst und die gewünschten Wirkungen werden erhalten.

Es hat sich überraschenderweise herausgestellt, daß die Verwendung mindestens einer erfindungsgemäßen Kombination mit einem Gehalt an (A) Camitin und/oder mindestens einem Acylcarnitin oder jeweils deren Derivaten, in Kombination mit einer Verbindung oder mehreren Verbindungen aus der Gruppe B, gebildet von Biotin, Liponsäure, konjugierten Fettsäuren, Carnosin, Biochinonen, Phytofluen, Phytoen und Folsäure und jeweils deren Derivaten, in kosmetischen oder dermatologischen Zubereitungen zur Behandlung und Prophylaxe der Symptome der intrinsischen und/oder extrinsischen Alterung der Haut und Hautanhangsgebilde sowie zur Behandlung und Prophylaxe der schädlichen Auswirkungen ultravioletter Strahlung und anderer, entzündliche Reaktionen induzierender Noxen auf die Haut und Hautanhangsgebilde, für die Behandlung und Prophylaxe von endogen oder exogen bedingten Hauterscheinungen, insbesondere degenerativen und/oder entzündlichen Hauterscheinungen, für die Vorbehandlung und/oder Nachbehandlung bei hautchirurgischen Maßnahmen und für die Behandlung oder Prophylaxe von Juckreiz, den Nachteilen das Standes der Technik abhilft.

Gegenstand der Erfindung ist die Verwendung einer Verbindung oder mehrerer Verbindungen aus der Gruppe A, gebildet von Carnitin und/oder Acyl-Camitinen und jeweils deren Derivaten in Kombination mit einer Verbindung oder mehreren Verbindungen aus der Gruppe B, gebildet von Biotin, Liponsäure, konjugierten Fettsäuren, Carnosin, Biochinonen, Phytofluen, Phytoen und Folsäure und jeweils deren Derivaten, zur Herstellung kosmetischer oder dermatologischer Zubereitungen für die Behandlung oder prophylaktische Behandlung der Symptome der intrinsischen und/oder extrinsischen Alterung der Haut und Hautanhangsgebilde, sowie zur Behandlung und Prophylaxe der schädlichen Auswirkungen ultravioletter Strahlung und anderer, entzündliche Reaktionen induzierender Noxen auf die Haut und Hautanhangsgebilde, für die Behandlung und Prophylaxe von endogen oder exogen bedingten Hauterscheinungen, insbesondere degenerativen und/oder entzündlichen Hauterscheinungen, für die Vorbehandlung und/oder Nachbehandlung bei hautchirurgischen Maßnahmen und für die Behandlung oder Prophylaxe von Juckreiz, gegebenenfalls unter zusätzlicher Verwendung von einer Verbindung oder mehreren Verbindungen aus der Gruppe der Antioxidantien und/oder Energiestoffwechsel-Metaboliten, wobei Zubereitungen mit einem Gehalt an Acyl-Carnitinen oder deren Derivaten mindestens eine Verbindung aus einer Gruppe enthalten, die von Biotin, konjugierten Fettsäuren, Biochinonen, Phytofluen, Phytoen und jeweils deren Derivaten gebildet wird.

Gegenstand der Erfindung sind auch kosmetische oder dermatologische Zubereitungen mit einem Gehalt an einer Verbindung oder mehrerer Verbindungen aus der Gruppe A, gebildet von Carnitin und/oder Acyl-Carnitinen und jeweils deren Derivaten in Kombination mit einer Verbindung oder mehreren Verbindungen aus der Gruppe B, gebildet von Biotin, Liponsäure, konjugierten Fettsäuren, Carnosin, Biochinonen, Phytofluen, Phytoen und Folsäure und jeweils deren Derivaten, gegebenenfalls mit einem zusätzlichen Gehalt an einer Verbindung oder mehreren Verbindungen aus der Gruppe der Antioxidantien und/oder Energiestoffwechsel-Metaboliten, wobei Zubereitungen mit einem Gehalt an Acyl-Carnitinen oder deren Derivaten mindestens eine Verbindung aus einer Gruppe enthalten, die von Biotin, konjugierten Fettsäuren, Biochinonen, Phytofluen, Phytoen und jeweils deren Derivaten gebildet wird.

Gegenstand der Erfindung ist auch die Verwendung einer Verbindung oder mehrerer Verbindungen aus der Gruppe A, gebildet von Carnitin und/oder Acyl-Camitinen und jeweils deren Derivaten in Kombination mit einer Verbindung oder mehreren Verbindungen aus der Gruppe B, gebildet von Biotin, Liponsäure, konjugierten Fettsäuren, Carnosin, Biochinonen, Phytofluen, Phytoen und Folsäure und jeweils deren Derivaten, für die Behandlung oder prophylaktische Behandlung der Symptome der intrinsischen und/oder extrinsischen Alterung der Haut und Hautanhangsgebilde sowie zur Behandlung und Prophylaxe der schädlichen Auswirkungen ultravioletter Strahlung und anderer, entzündliche Reaktionen induzierender Noxen auf die Haut und Hautanhangsgebilde, für die Behandlung und Prophylaxe von endogen oder exogen bedingten Hauterscheinungen, insbesondere degenerativen und/oder entzündlichen Hauterscheinungen, für die Vorbehandlung und/oder Nachbehandlung bei hautchirurgischen Maßnahmen und für die Behandlung oder Prophylaxe von Juckreiz, gegebenenfalls unter zusätzlicher Verwendung von einer Verbindung oder mehreren Verbindungen aus der Gruppe C, gebildet von Antioxidantien und/oder Energiestoffwechsel-Metaboliten, wobei die Verbindungen von A, B und gegebenenfalls C Bestandteile von Zubereitungen, insbesondere Bestandteile von kosmetischen oder dermatologischen Zubereitungen sind, und wobei Zubereitungen mit einem Gehalt an Acyl-Carnitinen oder deren Derivaten mindestens eine Verbindung aus einer Gruppe enthalten, die von Biotin, konjugierten Fettsäuren, Biochinonen, Phytofluen, Phytoen und jeweils deren Derivaten gebildet wird.

Bevorzugt werden topische Zubereitungen.

L-Carnitin [3-Hydroxy-4-(trimethylammonio)-buttersäurebetain], weist die Strukturformel 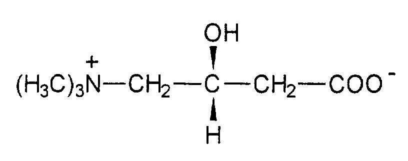 (Summenformel C₇H₁₅NO₃) auf.

Beide Enantiomere (D- oder L-Form) des Carnitins sind vorteilhaft im Sinne der vorliegenden Erfindung zu verwenden. Es kann auch von Vorteil sein, beliebige Enantiomerengemische, beispielsweise ein Racemat aus D- und L-Form, zu verwenden. Bevorzugt werden L-Camitin und/oder dessen Derivate.

Die L-Form des Carnitins ist in tierischen Geweben, insbesondere der gestreiften Muskulatur, weit verbreitet. Es dient im Fettsäure-Stoffwechsel als Überträger für Acylgruppen durch die Mitochondrien-Membran hindurch. Diese werden durch eine Acyltransferase von Acyl-Coenzym A auf die Hydroxy-Gruppe des L-Camitins übertragen. Der Transport von L-Camitin und Acyl-L-Carnitin durch die Membran erfolgt durch Vermittlung eines Transportproteins (Translocase).

Die Verwendung von nichtacyliertem Carnitin in kosmetischen oder dermatologischen Zubereitungen ist an sich bekannt, so beschreibt die FR-OS 2 654 618 die Verwendung von L-Carnitinderivaten in kosmetischen Zubereitungen zur Regulierung des Zellwachstums. Die US-PS 4,839,159 beschreibt topische Zubereitungen zur Verbesserung oder Prävention schädlicher Hautzustände, einschließlich der Faltenbildung, welche auf einen Verlust der Hautelastizität zurückzuführen ist.

Geeignete Derivate des Carnitins sind beispielsweise 0-Acylcarnitine mit geradkettigen oder verzweigten C₁-C₂₂-Alkylgruppen des Alkylcarbonylrestes (Acylrestes). Acetylcarnitin und dessen Derivate, z.B. wie nachstehend angegeben, werden bevorzugt. Carnitin und die Acylcarnitine können auch als Derivate wie beispielsweise Salze, Säureadditionssalze, Ester oder Amide verwendet werden.

Bevorzugt werden Acyl-Carnitine und deren Derivate gewählt aus der Gruppe der Substanzen der folgenden allgemeinen Strukturformel 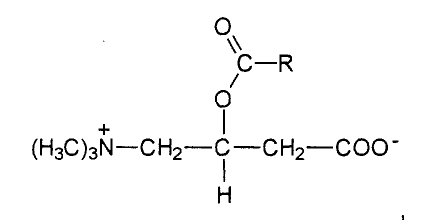 wobei R gewählt wird aus der Gruppe der verzweigten und unverzweigten Alkylreste mit bis zu 12 Kohlenstoffatomen. Bevorzugt sind Propionylcarnitin und, ganz besonders bevorzugt, Acetylcarnitin.

Beide Enantiomere (D- und L-Form) des Camitins oder der Acylcarnitine oder jeweils deren Derivate sind vorteilhaft im Sinne der vorliegenden Erfindung zu verwenden. Es kann auch von Vorteil sein, beliebige Enantiomerengemische, beispielsweise ein Racemat aus D- und L-Form, zu verwenden. Bevorzugt werden L-Acylcarnitine.

Bevorzugte Salze sind wasserlösliche Salze, z.B. Natrium-, Kalium- und Ammoniumsalze. Dies gilt auch für die Säureadditionssalze. Geeignete Säureadditionssalze werden mit anorganischen und organischen Säuren erhalten. Bevorzugt werden die Hydrochloride, Sulfate, Acetate, Caprylate oder Zitrate.

Geeignete Ester sind z.B. solche, die mit kurzkettigen, mittelkettigen oder langkettigen Alkoholen erhalten werden, vorzugsweise mono-Alkohole, insbesondere aber Methanol, Ethanol oder Propanol. Bevorzugt werden die Ethylester.

Bevorzugte Amide sind kurz- oder mittelkettige oder langkettige mono- und di-Alkylamide.

Alkyle der vorstehenden Substituenten enthalten z.B. bis zu 20, vorzugsweise bis zu 6 Kohlenstoffatome, insbesondere ein oder zwei Kohlenstoffatome.

Carnitin und/oder seine Derivate sind in den erfindungsgemäßen Zubereitungen bevorzugt in Mengen von 0,00001 bis 10 Gew.-%, insbesondere 0,01 bis 1,5 Gew.-%, jeweils bezogen auf das Gesamtgewicht der Zubereitungen, enthalten.

Als Biochinone werden unterschiedlich substituierte prenylierte Chinone bezeichnet, die in Menschen, Tieren und Pflanzen vorkommen.

Bevorzugte Biochinone sind Ubichinone, Plastochinone und Bovichinone, insbesondere aber Ubichinone.

Gut geeignete Ubichinone zeichnen sich durch die Strukturformel 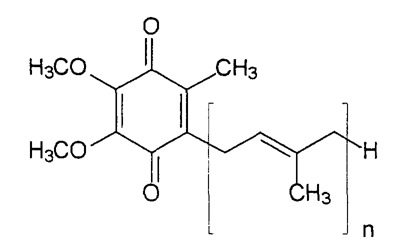 aus (n = 1-10) und stellen die am weitesten verbreiteten und damit am besten untersuchten Biochinone dar. Ubichinone werden je nach Zahl der in der Seitenkette verknüpften Isopren-Einheiten als Q-1, Q-2, Q-3 usw. oder nach Anzahl der C-Atome als U-5, U-10, U-15 usw. bezeichnet. Sie treten bevorzugt mit bestimmten Kettenlängen auf, z. B. in einigen Mikroorganismen u. Hefen mit n=6. Bei den meisten Säugetieren einschließlich des Menschen überwiegt Q-10.

Coenzym Q-10 wird bevorzugt. Es ist durch folgende Strukturformel gekennzeichnet:

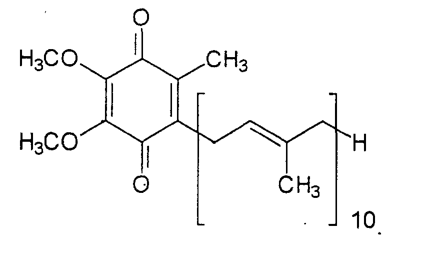

Ubichinone dienen den Organismen als Elektronenüberträger in der Atmungskette. Sie befinden sich in den Mitochondrien wo sie die cyclische Oxidation und Reduktion der Substrate des Citronensäure-Cyclus ermöglichen.

Gut geeignete Plastochinone weisen die allgemeine Strukturformel (n = 1-10) 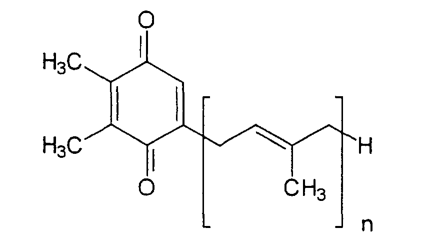 auf. Sie können aus Chloroplasten isoliert werden und spielen als Redoxsubstrate in der Photosynthese beim cyclischen und nichtcyclischen Elektronentransport eine Rolle, wobei sie reversibel in die entsprechenden Hydrochinone (Plastochinol) übergehen. Plastochinone unterscheiden sich in der Anzahl n der Isopren-Reste und werden endsprechend bezeichnet, z. B. PQ-9 (n=9). Ferner existieren andere Plastochinone mit unterschiedlichen Substituenten am Chinon-Ring.

Erfindungsgemäß bevorzugtes Biochinon ist das Coenzym Q-10.

Es ist vorteilhaft, in den fertigen Zubereitungen Konzentrationen von 0,00001 - 10 Gew.-%. Insbesondere 0,001 - 1,5 Gew.-%, an einem oder mehreren Biochinonen, bevorzugt Coenzym Q-10, zu wählen, jeweils bezogen auf das Gesamtgewicht der Zubereitungen.

Biotin (The Merck Index, 12. Auflage (1996), Abstract Nr. 1272) kann als Racemat oder in optisch aktiver Form (D- oder L-) vorliegen. Bevorzugt werden das in der Natur vorkommende D-Biotin und/oder dessen Derivate.

Geeignete Derivate des Biotins sind beispielsweise dessen Salze, Säureadditionssalze, Ester oder Amide.

Bevorzugte Salze von Biotin sind wasserlösliche Salze, z.B. Natrium-, Kalium- und Ammoniumsalze. Dies gilt auch für die Säureadditionssalze. Geeignete Säureadditionssalze werden mit anorganischen oder organischen Säuren erhalten. Bevorzugt werden die Hydrochloride, Sulfate, Acetate, Caprylate oder Zitrate.

Geeignete Ester des Biotins sind z.B. solche, die mit kurzkettigen oder mittelkettigen oder langkettigen Alkoholen erhalten werden, vorzugsweise mono-Alkohole, insbesondere aber Methanol, Ethanol oder Propanol. Bevorzugt werden die Ethylester.

Bevorzugte Amide sind kurz- oder mittelkettige oder langkettige mono- und di-Alkylamide.

Alkyle der vorstehenden Substituenten enthalten z.B. bis zu 20, vorzugsweise bis zu 6 Kohlenstoffatome, insbesondere ein oder zwei Kohlenstoffatome.

Biotin und/oder seine Derivate sind vorzugsweise in Mengen von 0,00001 bis 30 Gew.-%, besonders bevorzugt 0,01 bis 10 Gew.-%, insbesondere 01, - 7,5 Gew.-%, jeweils bezogen auf die gesamte Zubereitung, in den erfindungsgemäßen kosmetischen und dermatologischen Zubereitungen enthalten.

Liponsäure kann als Racemat oder in optisch aktiver Form (D- oder L-) vorliegen. Bevorzugt werden α-Liponsäure und/oder deren Derivate.

Geeignete Derivate der Liponsäure sind beispielsweise deren Salze, Säureadditionssalze, Ester oder Amide.

Bevorzugte Salze der Liponsäure sind wasserlösliche Salze, z.B. Natrium-, Kalium- und Ammoniumsalze. Dies gilt auch für die Säureadditionssalze. Geeignete Säureadditionssalze werden mit anorganischen oder organischen Säuren erhalten. Bevorzugt werden die Hydrochloride, Sulfate, Acetate, Caprylate oder Zitrate.

Geeignete Ester der Liponsäure sind z.B. solche, die mit kurzkettigen oder mittelkettigen oder langkettigen Alkoholen erhalten werden, vorzugsweise mono-Alkohole, insbesondere aber Methanol, Ethanol oder Propanol. Bevorzugt werden die Ethylester.

Bevorzugte Amide sind kurz- oder mittelkettige oder langkettige mono- und di-Alkylamide.

Alkyle der vorstehenden Substituenten enthalten z.B. bis zu 20, vorzugsweise bis zu 6 Kohlenstoffatome, insbesondere ein oder zwei Kohlenstoffatome.

Liponsäure und ihre Derivate sind vorzugsweise in Mengen von 0,00001 bis 30 Gew.-%, besonders bevorzugt 0,01 bis 10 Gew.-%, insbesondere 0,1 - 7,5 Gew.-%, jeweils bezogen auf die gesamte Zubereitung, in den erfindungsgemäßen kosmetischen und dermatologischen Zubereitungen enthalten.

Konjugierte Fettsäuren sind Monocarbonsäuren mit mindestens zwei konjugierten Mehrfachbindungen, insbesondere Doppelbindungen und deren Derivate. Sie werden hier auch "CFA" genannt. Gut geeignet sind alle geometrischen isomeren Formen und stellungsisomeren Formen sowie die Gemische solcher Verbindungen sowie deren Derivate, beispielsweise die Salze, Ester oder Amide.

Solche konjugierten Fettsäuren sind bekannt und nach bekannten Verfahren erhältlich, beispielsweise durch alkalische Isomerisierung der entsprechenden Fettsäuren mit isolierten Mehrfachbindungen bzw. Doppelbindungen.

Gut geeignete Fettsäuren können beispielsweise jeweils bis zu 24, vorzugsweise bis zu 18, insbesondere bis zu 12 Kohlenstoffatome besitzen und z.B. geradkettige oder verzweigte Alkyl-Monocarbonsäuren oder Cycloalkyl-Monocarbonsäuren sein. Sie können beispielsweise zwei bis sechs konjugierte Mehrfachbindungen, insbesondere Doppelbindungen besitzen.

Bevorzugte Salze sind wasserlösliche Salze, z.B. Natrium-, Kalium- und Ammoniumsalze.

Geeignete Ester sind z.B. solche, die mit kurzkettigen, mittelkettigen oder langkettigen Alkoholen erhalten werden, vorzugsweise Mono-Alkohole, insbesondere aber Methanol, Ethanol oder Propanol. Bevorzugt werden die Ethylester.

Bevorzugte Amide sind kurz- oder mittelkettige oder langkettige mono- und di-Alkylamide.

Alkyle der vorstehenden Substituenten enthalten z.B. bis zu 20, vorzugsweise bis zu 6 Kohlenstoffatome, insbesondere ein oder zwei Kohlenstoffatome.

Eine bevorzugte CFA, die z.B. den Energiestoffwechsel verbessert, ist die konjugierte Linolsäure, auch "CLA" genannt, in allen ihren geometrischen isomeren Formen und stellungsiosmeren Formen sowie den Gemischen solcher Verbindungen sowie ihren Derivaten, insbesondere wie vorstehend beschrieben.

Linolsäure (cis, cis-9,12-Octadecadiensäure) hat keine konjugierten Doppelbindungen. Distelöl und Sonnenblumenöl besitzen einen hohen Anteil an dieser Säure. Beispielsweise aus der Linolsäure dieser Rohstoffe erhält man durch alkalische Isomerisierung in bekannter Weise die konjugierten Verbindungen. Ein geeignetes Isomerengemisch ist auch in der Literatur beschrieben (Lipids, Vol. 34, Nr. 9 (1999), S. 997-1000, Tabelle 1). Bevorzugt liegen die konjugierten Doppelbindungen der CFAs im Bereich der Kohlenstoffatome 9 bis 12.

CFAs oder CLA und/oder die Derivate sind in den erfindungsgemäßen Zubereitungen bevorzugt in Mengen von 0,0001 bis 5 Gew.-%, insbesondere 0,01 bis 1,5 Gew.-%, jeweils bezogen auf das Gesamtgewicht der Zubereitungen, enthalten.

Carnosin kann als Racemat oder in optisch aktiver Form (D- oder L-) vorliegen. Bevorzugt werden L-Carnosin und/oder dessen Derivate.

Geeignete Derivate des Carnosins sind beispielsweise dessen Salze, Säureadditionssalze, Ester oder Amide.

Bevorzugte Salze des Carnosins sind wasserlösliche Salze, z.B. Natrium-, Kalium- und Ammoniumsalze. Dies gilt auch für die Säureadditionssalze. Geeignete Säureadditionssalze werden mit anorganischen oder organischen Säuren erhalten. Bevorzugt werden die Hydrochloride, Sulfate, Acetate, Caprylate oder Zitrate.

Geeignete Ester des Carnosins sind z.B. solche, die mit kurzkettigen oder mittelkettigen oder langkettigen Alkoholen erhalten werden, vorzugsweise mono-Alkohole, insbesondere aber Methanol, Ethanol oder Propanol. Bevorzugt werden die Ethylester.

Bevorzugte Amide sind kurz- oder mittelkettige oder langkettige mono- und di-Alkylamide.

Alkyle der vorstehenden Substituenten enthalten z.B. bis zu 20, vorzugsweise bis zu 6 Kohlenstoffatome, insbesondere ein oder zwei Kohlenstoffatome.

Carnosin und seine Derivate sind vorzugsweise in Mengen von 0,00001 bis 30 Gew.-%, besonders bevorzugt 0,01 bis 10 Gew.-%, insbesondere 0,1 -7,5 Gew.-%, jeweils bezogen auf die gesamte Zubereitung, in den erfindungsgemäßen kosmetischen und dermatologischen Zubereitungen enthalten.

Vorzugsweise wird Folsäure verwendet.

Folsäure und/oder ihre Derivate sind in den erfindungsgemäßen Zubereitungen bevorzugt in Mengen von 0,00001 bis 5 Gew.-%, insbesondere 0,01 bis 1,5 Gew.-%, jeweils bezogen auf das Gesamtgewicht der Zubereitungen, enthalten.

Geeignete Derivate der Folsäure sind beispielsweise deren Salze, Säureadditionssalze, Ester oder Amide.

Bevorzugte Salze der Folsäure sind wasserlösliche Salze, z.B. Natrium-, Kalium- und Ammoniumsalze. Dies gilt auch für die Säureadditionssalze. Geeignete Säureadditionssalze werden mit anorganischen oder organischen Säuren erhalten. Bevorzugt werden die Hydrochloride, Sulfate, Acetate, Caprylate oder Zitrate.

Geeignete Ester der Folsäure sind z.B. solche, die mit kurzkettigen oder mittelkettigen oder langkettigen Alkoholen erhalten werden, vorzugsweise mono-Alkohole, insbesondere aber Methanol, Ethanol oder Propanol. Bevorzugt werden die Ethylester.

Bevorzugte Amide sind kurz- oder mittelkettige oder langkettige mono- und di-Alkylamide.

Alkyle der vorstehenden Substituenten enthalten z.B. bis zu 20, vorzugsweise bis zu 6 Kohlenstoffatome, insbesondere ein oder zwei Kohlenstoffatome.
Phytofluen (Merck Index, 12. Auflage (1996), Zitat 7544) und Phytoen (Römpp ChemieLexikon (1991), Seite 3433) sind bekannt.

Phytoen un/oder Phytofluen können in den erfindungsgemäßen Zubereitungen bevorzugt in Mengen von 0,0001 bis 5 Gew.-%, insbesondere 0,01 bis 0,5 Gew.-%, jeweils bezogen auf das Gesamtgewicht der Zubereitungen enthalten sein.

Wirkstoff-Kombinationen mit einem oder mehreren Wirkstoffen der Gruppe A, vorzugsweise Carnitin und/oder seinen Derivaten, und einem oder mehreren Wirkstoffe aus der Gruppe B¹, gebildet von Biotin, Biochinonen, vorzugsweise Coenzym Q-10, Liponsäure und konjugierten Fettsäuren, vorzugsweise konjugierter Linolsäure, werden bevorzugt.

Besonders bevorzugt werden die Kombinationen der Wirkstoffe A, vorzugsweise Carnitin und/oder seinen Derivaten, mit Biotin und/oder konjugierten Fettsäuren, insbesondere konjugierter Linolsäure, oder mit Biotin und/oder Biochinonen, insbesondere Coenzym Q-10, wobei diese Kombinationen auch gemeinsam verwendet werden können. Jeweils können auch die Derivate verwendet werden.

Das Verhältnis der Gewichtsmengen der Kombinations-Wirkstoffe A/B kann in den Zubereitungen stark variieren. Beispielsweise kann es 1/10 bis 10/1, oder 5/1 bis 1/5 betragen. Vorzugsweise kann es aber auch 1/2 bis 2/1 und insbesondere 1/1 betragen.

Die erfindungsgemäßen Kombinationen mit Carnitin und Acylcarnitinen und jeweils deren Derivaten und den Kombinationspartnern werden im Rahmen dieser Schrift auch kollektiv als "erfindungsgemäßer Wirkstoff" oder "erfindungsgemäß verwendeteter Wirkstoff" bezeichnet bzw. mit sinnverwandten Bezeichnungen belegt. Dies gilt auch für die Verbindungen, die erfindungsgemäß als Kombinationspartner gewählt werden.

Mit dem Begriff "Haut" sind insbesondere die Körperhaut und die Kopfhaut gemeint. Eingeschlossen sind Talgdrüsen, Schweißdrüsen, dermale Kapillargefäße und die Haarwurzelbereiche.

Hautanhangsgebilde sind insbesondere Haare und Nägel.

Bei Anwendung des erfindungsgemäß verwendeten Wirkstoffes bzw. kosmetischer oder topischer dermatologischer Zubereitungen mit einem wirksamen Gehalt an erfindungsgemäß verwendetem Wirkstoff ist in überraschender Weise eine wirksame Behandlung, aber auch eine Prophylaxe
- von defizitären, sensitiven oder hypoaktiven Hautzuständen oder defizitären, sensitiven oder hypoaktiven Zustände von Hautanhangsgebilden
- von Erscheinungen vorzeitiger Alterung der Haut (z.B. Falten, Altersflecken, Teleangiektasien) und/oder der Hautanhangsgebilde,
- von umweltbedingten (Rauchen, Smog, reaktive Sauerstoffspezies, freie Radikale) und insbesondere lichtbedingten negativen Veränderungen der Haut und der Hautanhangsgebilde.
- von lichtbedingten Hautschäden
- von Pigmentierungsstörungen,
- von Juckreiz,
- von trockenen Hautzuständen und Hornschichtbarrierestörungen,
- von Haarausfall und für verbessertes Haarwachstum
- von entzündlichen Hautzuständen sowie atopischem Ekzem, seborrhoischem Ekzem, polymorpher Lichtdermatose, Psoriasis, Vitiligo
   möglich. Der erfindungsgemäße Wirkstoff bzw. kosmetischer oder topischer dermatologischer Zubereitungen mit einem wirksamen Gehalt an erfindungsgemäßem Wirkstoff dient aber auch in überraschender Weise
- zur Erhöhung der Hautfeuchtigkeit und zum Schutz vor erhöhtem transepidermalem Wasserverlust
- zur Beruhigung von empfindlicher oder gereizter Haut
- zur Stimulation der Kollagen-, Hyaluronsäure-, Elastinsynthese und Enzymaktivität
- zur Stimulation der intrazellulären DNA-Synthese, insbesondere bei defizitären oder hypoaktiven Hautzuständen
- zur Steigerung der Zellerneuerung und Regeneration der Haut
- zur Steigerung der hauteigenen Schutz- und Reparaturmechanismen (beispielsweise für dysfunktionelle Enzyme, DNA, Lipide, Proteine)
- zur Vor- und Nachbehandlung bei topischer Anwendung von Laser- und Abschleifbehandlungen, die z. B. der Reduzierung von Hautfalten und Narben dienen, um den resultierenden Hautreizungen entgegenzuwirken und die Regenerationsprozesse in der verletzten Haut zu fördern
- zum Schutz und zur Neubildung von dermalen Kapillargefäßen, insbesondere bei Altershaut.

Es ist erfindungsgemäß insbesondere äußerst vorteilhaft, den erfindungsgemäß verwendeten Wirkstoff bzw. kosmetische oder topische dermatologische Zubereitungen mit einem wirksamen Gehalt an erfindungsgemäß verwendetem Wirkstoff zur kosmetischen oder dermatologischen Behandlung oder Prophylaxe unerwünschter Hautzustände zu verwenden.

Erfindungsgemäß können in Zubereitungen, welche die erfindungsgemäßen Wirkstoffkombinationen enthalten, übliche Antioxidantien eingesetzt werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Carotinoide, Carotine (z.B. α-Carotin, β-Carotin, Lycopin) und deren Derivate, Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Alanindiessigsäure, Flavonoide, Polyphenole, Catechine, Vitamin C und Derivate (z.B. Ascorbylpalmitat, Mg-Ascorbylphosphat, Ascorbylacetat), Tocopherole und Derivate (z.B. Vitamin-E-acetat), sowie Koniferylbenzoat des Benzoëharzes, Flavonoide, z.B. alpha-Glucosylrutin, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄), Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantjen (eine oder mehrere Verbindungen) in den Zubereitungen beträgt vorzugsweise 0,001 bis 30 Gew.-%, besonders bevorzugt 0,05 - 20 Gew.-%, insbesondere 1- 10 Gew.-%, bezogen auf das Gesamtgewicht der Zubereitung.

Geeignete Energiestoffwechsel-Metaboliten sind insbesondere Cholin, Adenin oder Adenosin.

Die Menge der Energiestoffwechsel-Metaboliten (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Das Gewichtsverhältnis der Gesamtmenge an Carnitinen und/oder Acylcarnitinen zur Gesamtmenge (jeweils Gewichtsmengen) an Verbindungen der Gruppe B wird vorteilhaft aus dem Bereich von 10 : 1 bis 1 : 10, bevorzugt 5 : 1 bis 1 : 5, insbesondere bevorzugt 1 : 2 bis 2 : 1 gewählt.

Die Prophylaxe bzw. die kosmetische oder dermatologische Behandlung mit dem erfindungsgemäß verwendeten Wirkstoff bzw. mit den kosmetischen oder topischen dermatologischen Zubereitungen mit einem wirksamen Gehalt an erfindungsgemäß verwendetem Wirkstoff erfolgt in der üblichen Weise, und zwar dergestalt, daß der erfindungsgemäß verwendete Wirkstoff bzw. die kosmetischen oder topischen dermatologischen Zubereitungen mit einem wirksamen Gehalt an erfindungsgemäß verwendetem Wirkstoff auf die betroffenen Hautstellen oder die Haaranhangsgebilde aufgetragen wird.

Vorteilhaft kann der erfindungsgemäß verwendete Wirkstoff eingearbeitet werden in übliche kosmetische und dermatologische Zubereitungen, welche in verschiedenen Formen vorliegen können. So können sie z.B. eine Lösung, eine Emulsion vom Typ Wasser-in-Öl (W/O) oder vom Typ Öl-in-Wasser (O/W), oder eine multiple Emulsionen, beispielsweise vom Typ Wasser-in-Öl-in-Wasser (W/O/W) oder Öl-in-Wasser-in-Öl (O/W/O), eine Hydrodispersion oder Lipodispersion, ein Gel, einen festen Stift oder auch ein Aerosol darstellen.

Erfindungsgemäße Emulsionen im Sinne der vorliegenden Erfindung, z.B. in Form einer Creme, einer Lotion, einer kosmetischen Milch sind vorteilhaft und enthalten z.B. Fette, Öle, Wachse und/oder andere Fettkörper, sowie Wasser und einen oder mehrere Emulgatoren, wie sie üblicherweise für einen solchen Typ der Formulierung verwendet werden.

Es ist auch möglich und vorteilhaft im Sinne der vorliegenden Erfindung, den erfindungsgemäß verwendeten Wirkstoff in wäßrige Systeme bzw. Tensidzubereitungen zur Reinigung der Haut und der Haare einzufügen.

Es ist dem Fachmanne natürlich bekannt, daß anspruchsvolle kosmetische Zusammensetzungen zumeist nicht ohne die üblichen Hilfs- und Zusatzstoffe denkbar sind. Darunter zählen beispielsweise Konsistenzgeber, Füllstoffe, Parfum, Farbstoffe, Emulgatoren, zusätzliche Wirkstoffe wie Vitamine oder Proteine, Lichtschutzmittel, Stabilisatoren, Insektenrepellentien, Alkohol, Wasser, Salze, antimikrobiell, proteolytisch oder keratolytisch wirksame Substanzen usw.

Mutatis mutandis gelten entsprechende Anforderungen an die Formulierung medizinischer Zubereitungen.

Medizinische topische Zusammensetzungen im Sinne der vorliegenden Erfindung enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Es ist dabei ebenfalls von Vorteil, den erfindungsgemäß verwendeten Wirkstoff als Zusatzstoff zu Zubereitungen zu geben, die bereits andere Wirkstoffe für andere Zwecke enthalten.

Entsprechend können kosmetische oder topische dermatologische Zusammensetzungen im Sinne der vorliegenden Erfindung, je nach ihrem Aufbau, beispielsweise verwendet werden als Hautschutzcrème, Reinigungsmilch, Sonnenschutzlotion, Nährcrème, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Günstig sind gegebenenfalls auch solche kosmetischen und dermatologischen Zubereitungen, die in der Form eines Sonnenschutzmittels vorliegen. Vorzugsweise enthalten diese neben dem erfindungsgemäß verwendeten Wirkstoff zusätzlich mindestens eine UVA-Filtersubstanz und/oder mindestens eine UVB-Filtersubstanz und/oder mindestens ein anorganisches Pigment.

Es ist aber auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden beispielsweise in Tagescrèmes gewöhnlich UV-A- bzw. UV-B-Filtersubstanzen eingearbeitet.

Vorteilhaft können erfindungsgemäße Zubereitungen Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen.

Die UVB-Filter können öllöslich oder wasserlöslich sein. Als öllösliche Substanzen sind z.B. zu nennen:
- 3-Benzylidencampher und dessen Derivate, z.B. 3-(4-Methylbenzyliden)campher,
- 4-Aminobenzoësäure-Derivate, vorzugsweise 4-(Dimethylamino)-benzoësäure(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;
- 2,4,6-Trianilino-(p-carbo-2'-ethyl-1'-hexyloxy)-1,3,5-triazin.

Als wasserlösliche Substanzen sind vorteilhaft:
- 2-Phenylbenzimidazol-5-sulfonsäure und deren Salze, z.B. Natrium-, Kalium- oder Triethanolammonium-Salze,
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Die Liste der genannten UVB-Filter, die erfindungsgemäß Verwendung finden können, soll selbstverständlich nicht limitierend sein.

Gegenstand der Erfindung ist auch die Kombination eines erfindungsgemäßen UVA-Filters mit einem UVB-Filter bzw. eine erfindungsgemäßes kosmetische oder dermatologische Zubereitung, welche auch einen UVB-Filter enthält.

Es kann auch von Vorteil sein, in erfindungsgemäßen Zubereitungen UVA-Filter einzusetzen, die üblicherweise in kosmetischen und/oder dermatologischen Zubereitungen enthalten sind. Bei solchen Filtersubstanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch Zubereitungen, die diese Kombinationen enthalten, sind Gegenstand der Erfindung. Es können die gleichen Mengen an UVA-Filtersubstanzen verwendet werden, welche für UVB-Filtersubstanzen genannt wurden.

Kosmetische und/oder dermatologische Zubereitungen im Sinne der vorliegenden Erfindung können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid. Es können die für die vorstehenden Kombinationen genannten Mengen verwendet werden.

Die erfindungsgemäßen kosmetischen und dermatologischen Zubereitungen können kosmetische Wirk-, Hilfs- und/oder Zusatzstoffe enthalten, wie sie üblicherweise in solchen Zubereitungen verwendet werden, z.B. Antioxidationsmittel, Konservierungsmittel, Bakterizide, Parfüme, Substanzen zum Verhindern des Schäumens, Farbstoffe, Pigmente, die färbende Wirkung haben, Verdickungsmittel, oberflächenaktive Substanzen, Emulgatoren, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Sofern die kosmetische oder dermatologische Zubereitung im Sinne der vorliegenden Erfindung eine Lösung oder Emulsion oder Dispersion darstellt, können als Lösungsmittel verwendet werden:
- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl,-monoethyl- oder -monobutylether, Diethylenglykolmonomethyl- oder-monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkybenzoat und 2-Ethylhexylisostearat, Mischungen aus C₁₂₋₁₅-Alkybenzoat und Isotridecylisononanoat sowie Mischungen aus C₁₂₋₁₅-Alkybenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wäßrige Phase der erfindungsgemäßen Zubereitungen enthält gegebenenfalls vorteilhaft
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, - monoethyl- oder -monobutylether, Diethylenglykolmonomethyl- oder - monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Erfindungsgemäß verwendete Gele enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Feste Stifte enthalten z.B. natürliche oder synthetische Wachse, Fettalkohole oder Fettsäureester.

Übliche Grundstoffe, welche für die Verwendung als kosmetische Stifte im Sinne der vorliegenden Erfindung geeignet sind, sind flüssige Öle (z.B. Paraffinöle, Ricinusöl, Isopropylmyristat), halbfeste Bestandteile (z.B. Vaseline, Lanolin), feste Bestandteile (z.B. Bienenwachs, Ceresin und Mikrokristalline Wachse bzw. Ozokerit) sowie hochschmelzende Wachse (z.B. Carnaubawachs, Candelillawachs)

Als Treibmittel für aus Aerosolbehältern versprühbare kosmetische und/oder dermatologische Zubereitungen im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fiuorkohlenwasserstoffe und Fluorchlorkohlenwasserstoffe (FCKW).

Kosmetische Zubereitungen im Sinne der vorliegenden Erfindung können auch als Gele vorliegen, die neben einem wirksamen Gehalt am erfindungsgemäßen Wirkstoff und dafür üblicherweise verwendeten Lösungsmitteln, bevorzugt Wasser, noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise Bentonite, oder ein Gemisch aus Polyethylenglykol und Polyethylenglykolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Alle Mengenangaben, Anteile und Prozentanteile sind, soweit nicht anders angegeben, auf das Gewicht und die Gesamtmenge bzw. auf das Gesamtgewicht der Zubereitungen bezogen.

Die nachfolgenden Beispiele sollen die vorliegende Erfindung verdeutlichen, ohne sie einzuschränken. Es werden Gewichtsprozente angegeben.

CLA1 bedeutet in den folgenden Beispielen die folgende Fettsäure-CLA-lsomeren-Zubereitung:

**Tabelle 1:**

| CLA1 | |
|---|---|
| Fettsäure | Gew.-% |
| 16:0 | 6,9 |
| 18:0 | 2,5 |
| 18 : 1 | 15,3 |
| 18:2 | 0,8 |
| 18 : 2 (CLA) | 73,8 a) |
| (Rest nicht definiert) | |

| a) CLA-Zusammensetzung (- Octadiensäure) | |
|---|---|
| | Gew.-% |
| 9c, 11t/9t, 11c- | 34,6 |
| 10t, 12c- | 35,9 |
| 9c, 11c/10c, 12c- | 1,7 |
| 9t, 11t/10t, 12t- | 1,6 |

| Beispiel 1 | |
|---|---|
| W/O Creme | |
| | Gew.% |
| Paraffinöl (DAB 9) | 10,00 |
| Petrolatum | 4,00 |
| Wollwachsalkohol | 1,00 |
| PEG-7-Hydriertes Rizinusöl | 3,00 |
| Aluminiumstearat | 0,40 |
| Phytoen | 0,08 |
| D-Biotin | 0,04 |
| Glycerin | 2,00 |
| Konservierungsmittel, Farbstoffe, Parfüm | q.s. |
| O-Propionyl-L-Carnitin | 0,20 |
| Wasser | ad 100,00 |

| Beispiel 2 | |
|---|---|
| W/O Lotion | |
| | Gew.-% |
| Paraffinöl (DAB 9) | 20,00 |
| Petrolatum | 4,00 |
| Glucosesesquiisostearat | 2,00 |
| Aluminiumstearat | 0,40 |
| α-Tocopherylacetat | 1,00 |
| Glycerin | 5,00 |
| Konservierungsmittel, Farbstoffe, Parfüm | q.s. |
| Coenzym Q10 | 0,05 |
| O-Acetyl-L-Carnitin | 2,50 |
| Wasser | ad 100,00 |

| Beispiel 3 | |
|---|---|
| O/W Lotion | |
| | Gew.-% |
| Paraffinöl (DAB 9) | 8,00 |
| Isopropylpalmitat | 3,00 |
| Petrolatum | 4,00, |
| Cetylstearylalkohol | 2,00 |
| PEG 40 Rizinusöl | 0,50 |
| Natriumcetylstearylsulfat | 0,50 |
| Natrium Carbomer | 0,40 |
| Glycerin | 3,00 |
| | |
| Folsäure | 0,50 |
| Biotin | 0,20 |
| Octylmethoxycinnamat | 5,00 |
| Butylmethoxydibenzoylmethan | 1,00 |
| Konservierungsmittel, Farbstoffe, Parfüm | q.s. |
| Carnitin | 3,00 |
| Wasser | ad 100,00 |

| Beispiel 4 | |
|---|---|
| O/W Creme | |
| | Gew.-% |
| Paraffinöl (DAB 9) | 7,00 |
| Avocadoöl | 4,00 |
| Glycerylmonostearat | 2,00 |
| Natriumlactat | 3,00 |
| Glycerin | 3,00 |
| Phytofluen | 0,01 |
| Coenzym Q10 | 0,003 |
| Konservierungsmittel, Farbstoffe, Parfüm | q.s. |
| Carnitin | 3,00 |
| Wasser | ad 100,00 |

| Beispiel 5 | |
|---|---|
| Liposomenhaltiges Gel | |
| | Gew.-% |
| Lecithin | 6,00 |
| Schibutter | 3,00 |
| α-Glucosylrutin | 0,20 |
| Natriumcitrat | 0,50 |
| Glycin | 0,20 |
| Harnstoff | 0,20 |
| Natrium PCA | 0,50 |
| Hydrolysiertes Kollagen | 2,00 |
| Xanthan Gummi | 1,40 |
| Sorbitol | 3,00 |
| Konservierungsmittel, Farbstoffe, Parfüm | q.s. |
| O-Acetyl-L-Carnitin | 1,00 |
| Vitamin C | 0,50 |
| Biotin | 0,004 |
| Wasser | ad 100,00 |

| Beispiel 6 | |
|---|---|
| Sonnenschutzemulsion | |
| | Gew.-% |
| Cyclomethicon | 2,00 |
| Cetyldimethicon Copolyol | 0,20 |
| PEG 22-Dodecyl Copolymer | 3,00 |
| Paraffinöl (DAB 9) | 2,00 |
| Caprylsäure-/Caprinsäure Triglycerid | 5,80 |
| Octylmethoxycinnamat | 5,80 |
| Butyl-methoxy-dibenzoylmethan | 4,00 |
| O-Acetyl-DL-Carnitin | 0,10 |
| L-Carnitin | 0,50 |
| Phytoen | 0,80 |
| α-Tocopherylacetat | 0,50 |
| ZnSO₄ | 0,70 |
| Na₄EDTA | 0,30 |
| Konservierungsmittel, Farbstoffe, Parfüm | q.s. |
| Wasser | ad 100,00 |

| Beispiel 7 | |
|---|---|
| Sonnenschutzemulsion | |
| | Gew.-% |
| Cyclomethicon | 2,00 |
| Cetylstearylalkohol + PEG 40-hydriertes Rizinusöl + Natrium Cetylstearylsulfat | 2,50 |
| Glyceryllanolat | 1,00 |
| Caprylsäure-/Caprinsäure Triglycerid | 0,10 |
| Laurylmethicon Copolyol | 2,00 |
| Octylstearat | 3,00 |
| Rizinusöl | 4,00 |
| Glycerin | 3,00 |
| Acrylamid/Natriumacrylat Copolymer | 0,30 |
| Hydroxypropylmethylcellulose | 0,30 |
| Octylmethoxycinnamat | 5,00 |
| Butyl-methoxy-dibenzoylmethan | 0,50 |
| L-Carnitin | 0,20 |
| D-Biotin | 0,02 |
| Alpha Liponsäure | 0,30 |
| α-Tocopherylacetat | 1,00 |
| Na₃HEDTA | 1,50 |
| Konservierungsmittel, Farbstoffe, Parfüm | q.s. |
| Wasser | ad 100,00 |

| Beispiel 8 | |
|---|---|
| Sonnenschutzemulsion | |
| | Gew.-% |
| Cyclomethicone | 2,00 |
| Cetylstearylalkohol +PEG 40-hydriertes Rizinusöl +Natrium Cetylstearylsulfat | 2,50 |
| Glyceryllanolat | 1,00 |
| Caprylsäure-/Caprinsäure Triglycerid | 0,10 |
| Laurylmethicon Copolyol | 2,00 |
| Octylstearat | 3,00 |
| Rizinusöl | 4,00 |
| Glycerin | 3,00 |
| Acrylamid/Natriumacrylat Copolymer | 0,30 |
| Hydroxypropylmethylcellulose | 0,30 |
| Octylmethoxycinnamat | 5,00 |
| Butyl-methoxy-dibenzoylmethan | 0,75 |
| Na₃HEDTA | 1,50 |
| Konservierungsmittel, Farbstoffe, Parfüm | q.s. |
| L-Carnitin | 3,00 |
| CLA 1 | 0,05 |
| Wasser | ad 100,00 |

| Beispiel 9 | |
|---|---|
| Sprayformulierung | |
| | Gew.-% |
| α-Tocopherol | 0,10 |
| O-Acetyl-DL-Carnitin | 0,80 |
| CLA 1 | 0,50 |
| Ethanol | 28,20 |
| Konservierungsmittel, Farbstoffe, Parfüm | q.s. |
| Propan/Butan 25/75 | ad 100,00 |

| Beispiel 10 | |
|---|---|
| Haarregenerationskur | |
| | Gew.-% |
| Bis-Diglycerylpolyacyladipat-2 | 3,00 |
| Carnitin | 0,80 |
| Behenylalkohol | 4,20 |
| Coenzym Q10 | 0,03 |
| Alpha Glucosylrutin | 0,10 |
| NaOH 45%-ig | 0,16 |
| CLA 1 | 0,04 |
| D-Biotin | 0,04 |
| Na₂H₂EDTA | 0,20 |
| Citronensäure | 0,50 |
| Cetrimoniumchlorid | 5,00 |
| Adenosin | 0,10 |
| Cholin | 0,20 |
| Konservierungsmittel, Farbstoffe, Parfüm | q.s. |
| Wasser | ad 100,00 |

| Beispiel 11 | |
|---|---|
| Haarpflegekur | |
| | Gew.-% |
| Bis-Diglycerylpolyacyladipat-2 | 3,00 |
| O-Acetyl-DL-Carnitin | 0,50 |
| Behenylalkohol | 4,20 |
| Coenzym Q10 | 0,01 |
| L-Carnosine | 0,20 |
| NaOH 45%-ig | 0,16 |
| D-Biotin | 0,04 |
| Na₂H₂EDTA | 0,20 |
| Citronensäure | 0,60 |
| Cetrimoniumchlorid | 5,00 |
| Alpha Tocopheryl -acetat | 0,10 |
| Konservierungsmittel, Farbstoffe, Parfüm | q.s. |
| Wasser | ad 100,00 |

| **Beispiele 12- 14** | | | |
|---|---|---|---|
| Conditioner-Shampoo mit Perlglanz | | | |
| | **1** | **2** | **3** |
| Polyquaternium-10 | 0,5 | 0,5 | 0,5 |
| Natriumlaurethsulfat | 9,0 | 9,0 | 9,0 |
| Cocoamidopropylbetain | 2,5 | 2,5 | 2,5 |
| Perlglanzmittel | 2,0 | 2,0 | 2,0 |
| Gamma-Oryzanol | 0,01 | 0,15 | 0,05 |
| O-Acetylcarnitin | 0,001 | 0,05 | 0,1 |
| L-Carnitin | 0,03 | 0,01 | 0,005 |
| Phytoen | 0,001 | 0,001 | 0,005 |
| Calziumlactat | 0,15 | 0,05 | 0,1 |
| Konservierungsmittel, Parfüm, Verdicker, pH-Einstellung und Lösungsvermittler | q.s. | q.s. | q.s. |
| Wasser, VES (vollentsalzt) | ad 100,0 | ad 100,0 | ad 100,0 |

Der pH-Wert wird auf 6 eingestellt.

## Patentansprüche

1. Kosmetische oder dermatologische Zubereitungen mit einem Gehalt an einer Verbindung oder mehrerer Verbindungen aus der Gruppe A, gebildet von Carnitin und/oder Acyl-Camitinen und jeweils deren Derivaten in Kombination mit einer Verbindung oder mehreren Verbindungen aus der Gruppe B, gebildet von Biotin, Liponsäure, konjugierten Fettsäuren, Carnosin, Biochinonen, Phytofluen, Phytoen und Folsäure und jeweils deren Derivaten, gegebenenfalls mit einem zusätzlichen Gehalt an einer Verbindung oder mehreren Verbindungen aus der Gruppe der Antioxidantien und/oder Energiestoffwechsel-Metaboliten, wobei Zubereitungen mit einem Gehalt an Acyl-Camitinen oder deren Derivaten mindestens eine Verbindung aus einer Gruppe enthalten, die von Biotin, konjugierten Fettsäuren, Biochinonen, Phytofluen, Phytoen und jeweils deren Derivaten gebildet wird.

2. Verwendung einer Verbindung oder mehrerer Verbindungen aus der Gruppe A, gebildet von Carnitin und/oder Acyl-Carnitinen und jeweils deren Derivaten in Kombination mit einer Verbindung oder mehreren Verbindungen aus der Gruppe B, gebildet von Biotin, Liponsäure, konjugierten Fettsäuren, Carnosin, Biochinonen, Phytofluen, Phytoen und Folsäure und jeweils deren Derivaten, für die Behandlung oder prophylaktische Behandlung der Symptome der intrinsischen und/oder extrinsischen Alterung der Haut und Hautanhangsgebilde sowie zur Behandlung und Prophylaxe der schädlichen Auswirkungen ultravioletter Strahlung und anderer, entzündliche Reaktionen induzierender Noxen auf die Haut und Hautanhangsgebilde, für die Behandlung und Prophylaxe von endogen oder exogen bedingten Hauterscheinungen, insbesondere degenerativen und/oder entzündlichen Hauterscheinungen, für die Vorbehandlung und/oder Nachbehandlung bei hautchirurgischen Maßnahmen und für die Behandlung oder Prophylaxe von Juckreiz, gegebenenfalls unter zusätzlicher Verwendung von einer Verbindung oder mehreren Verbindungen aus der Gruppe C, gebildet von Antioxidantien und/oder Energiestoffwechsel-Metaboliten, wobei die Verbindungen von A, B und gegebenenfalls C Bestandteile von Zubereitungen, insbesondere Bestandteile von kosmetischen oder dermatologischen Zubereitungen sind, und wobei Zubereitungen mit einem Gehalt an Acyl-Camitinen oder deren Derivaten mindestens eine Verbindung aus einer Gruppe enthalten, die von Biotin, konjugierten Fettsäuren, Biochinonen, Phytofluen, Phytoen und jeweils deren Derivaten gebildet wird.

3. Verwendung einer Verbindung oder mehrerer Verbindungen aus der Gruppe A, gebildet von Carnitin und/oder Acyl-Carnitinen und jeweils deren Derivaten in Kombination mit einer Verbindung oder mehreren Verbindungen aus der Gruppe B, gebildet von Biotin, Liponsäure, konjugierten Fettsäuren, Carnosin, Biochinonen, Phytofluen, Phytoen und Folsäure und jeweils deren Derivaten, zur Herstellung kosmetischer oder dermatologischer Zubereitungen für die Behandlung oder prophylaktische Behandlung der Symptome der intrinsischen und/oder extrinsischen Alterung der Haut und Hautanhangsgebilde, sowie zur Behandlung und Prophylaxe der schädlichen Auswirkungen ultravioletter Strahlung und anderer, entzündliche Reaktionen induzierender Noxen auf die Haut und Hautanhangsgebilde, für die Behandlung und Prophylaxe von endogen oder exogen bedingten Hauterscheinungen, insbesondere degenerativen und/oder entzündlichen Hauterscheinungen, für die Vorbehandlung und/oder Nachbehandlung bei hautchirurgischen Maßnahmen und für die Behandlung oder Prophylaxe von Juckreiz, gegebenenfalls unter zusätzlicher Verwendung von einer Verbindung oder mehreren Verbindungen aus der Gruppe der Antioxidantien und/oder Energiestoffwechsel-Metaboliten, wobei Zubereitungen mit einem Gehalt an Acyl-Camitinen oder deren Derivaten mindestens eine Verbindung aus einer Gruppe enthalten, die von Biotin, konjugierten Fettsäuren, Biochinonen, Phytofluen, Phytoen und jeweils deren Derivaten gebildet wird.

4. Verwendung gemäß Anspruch 2 oder 3, **dadurch gekennzeichnet, dass** die Behandlung oder prophylaktische Behandlung
- von defizitären, sensitiven oder hypoaktiven Hautzuständen oder defizitären, sensitiven oder hypoaktiven Zustände von Hautanhangsgebilden
- von Erscheinungen vorzeitiger Alterung der Haut (z.B. Falten, Altersflecken, Teleangiektasien) und/oder der Hautanhangsgebilde,
- von umweltbedingten (Rauchen, Smog, reaktive Sauerstoffspezies, freie Radikale) und insbesondere lichtbedingten negativen Veränderungen der Haut und der Hautanhangsgebilde.
- von lichtbedingten Hautschäden
- von Pigmentierungsstörungen,
- von Juckreiz,
- von trockenen Hautzuständen und Homschichtbarrierestörungen,
- von Haarausfall und für verbessertes Haarwachstum
- von entzündlichen Hautzuständen sowie atopischem Ekzem, seborrhoischem Ekzem, polymorpher Lichtdermatose, Psoriasis, Vitiligo erfolgt und
- zur Erhöhung der Hautfeuchtigkeit und zum Schutz vor erhöhtem transepidermalem Wasserverlust
- zur Beruhigung von empfindlicher oder gereizter Haut
- zur Stimulation der Kollagen-, Hyaluronsäure-, Elastinsynthese und Enzymaktivität
- zur Stimulation der intrazellulären DNA-Synthese, insbesondere bei defizitären oder hypoaktiven Hautzuständen
- zur Steigerung der Zellerneuerung und Regeneration der Haut
- zur Steigerung der hauteigenen Schutz- und Reparaturmechanismen (beispielsweise für dysfunktionelle Enzyme, DNA, Lipide, Proteine)
- zur Vor- und/oder Nachbehandlung bei topischer Anwendung von Laser-und Abschleifbehandlungen, die z. B. der Reduzierung von Hautfalten und Narben dienen, um insbesondere den resultierenden Hautreizungen entgegenzuwirken und die Regenerationsprozesse in der verletzten Haut zu fördern
- zum Schutz und zur Neubildung von dermalen Kapillargefäßen, insbesondere bei Altershaut, dient.

5. Zubereitungen gemäß Anspruch 1, **gekennzeichnet durch** den Gehalt an einer Wirkstoff-Kombinationen mit einem oder mehreren Wirkstoffen der Gruppe A, vorzugsweise Carnitin und/oder seinen Derivaten, und einem oder mehreren Wirkstoffe aus der Gruppe B¹, gebildet von Biotin, Biochinonen, vorzugsweise Coenzym Q-10, Liponsäure und konjugierten Fettsäuren, vorzugsweise konjugierter Linolsäure.

6. Zubereitungen gemäß Anspruch 1, **gekennzeichnet durch** einen Gehalt einer Kombinationen der Wirkstoffe A, vorzugsweise Carnitin und/oder seinen Derivaten, mit Biotin und/oder konjugierten Fettsäuren, insbesondere konjugierter Linolsäure, oder mit Biotin und/oder Biochinonen, insbesondere Coenzym Q-10.

7. Zubereitungen gemäß Anspruch 1, **gekennzeichnet durch** einen Gehalt an den Wirkstoffkombinationen von Anspruch 2 und 3.

8. Verwendung gemäß Anspruch 2 oder von Zubereitungen mit Wirkstoffkombinationen gemäß den Ansprüchen 5 und 6.
